(19) Europäisches Patentamt / European Patent Office / Office européen des brevets

(11) **EP 4 192 818 B1**

(12) **EUROPEAN PATENT SPECIFICATION**

(45) Date of publication and mention
of the grant of the patent:
**09.10.2024 Bulletin 2024/41**

(21) Application number: **21759038.9**

(22) Date of filing: **04.08.2021**

(51) International Patent Classification (IPC):
**B01J 23/14** (2006.01)    **B01J 29/65** (2006.01)
**B01J 35/61** (2024.01)    **C01B 39/02** (2006.01)
**C01B 39/36** (2006.01)    **C01B 39/40** (2006.01)
**C07D 239/06** (2006.01)

(52) Cooperative Patent Classification (CPC):
**C07D 239/06; B01J 23/14; B01J 29/65;
B01J 29/655; B01J 35/615; B01J 35/617;
C01B 39/026; C01B 39/36; C01B 39/40;**
B01J 2229/42

(86) International application number:
**PCT/EP2021/071729**

(87) International publication number:
**WO 2022/029154 (10.02.2022 Gazette 2022/06)**

(54) **HETEROGENEOUS CATALYZED PROCESS FOR THE PRODUCTION OF 2,2,4,6,6-PENTAMETHYL-1,2,5,6-TETRAHYDRO-PYRIMIDINE**

HETEROGENES KATALYSIERTES VERFAHREN ZUR HERSTELLUNG VON 2,2,4,6,6-PENTAMETHYL-1,2,5,6-TETRAHYDRO-PYRIMIDIN

PROCÉDÉ CATALYSÉ HÉTÉROGÈNE POUR LA PRODUCTION DE 2,2,4,6,6-PENTAMÉTHYL-1,2,5,6-TETRAHYDRO-PYRIMIDINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.08.2020 EP 20189315**

(43) Date of publication of application:
**14.06.2023 Bulletin 2023/24**

(73) Proprietor: **BASF SE**
**67056 Ludwigshafen am Rhein (DE)**

(72) Inventors:
• **HEYDT, Thomas**
**67056 Ludwigshafen am Rhein (DE)**
• **GORDILLO BOLONIO, Alvaro**
**08017 Barcelona (ES)**
• **SCHMITT, Michael**
**67056 Ludwigshafen am Rhein (DE)**
• **GUENTHER, Klaus**
**40037 Pontecchio Marconi BO (IT)**
• **MÜLLER, Ulrich**
**67435 Neustadt (DE)**
• **PARVULESCU, Andrei-Nicolae**
**67056 Ludwigshafen am Rhein (DE)**
• **KUEBLER, Michael**
**67056 Ludwigshafen am Rhein (DE)**

(74) Representative: **BASF IP Association**
**BASF SE**
**GBI-C006**
**67056 Ludwigshafen (DE)**

(56) References cited:
**WO-A1-2015/059171     DE-B- 1 276 041**

• **GLIOZZI GHERARDO ET AL: "Towards a more sustainable production of triacetoneamine with heterogeneous catalysis", JOURNAL OF MOLECULAR CATALYSIS A: CHEMICAL, vol. 393, 26 June 2014 (2014-06-26), pages 325 - 332, XP029041319, ISSN: 1381-1169, DOI: 10.1016/J.MOLCATA.2014.06.023**

- **JUN TIAN ET AL: "Effect of Alkali Treatment of HY Zeolite on Continuous Synthesis of Triacetonamine", JOURNAL OF HETEROCYCLIC CHEMISTRY, vol. 52, no. 5, 31 July 2014 (2014-07-31), US, pages 1377 - 1381, XP055551991, ISSN: 0022-152X, DOI: 10.1002/jhet.2242**
- **VOLODARSKY L ET AL: "Synthesis of acetonine nitroxide radical and 1-hydroxy-2,2,4,6,6-pentamethyl-1,2,5,6-tetrahy dropyrimidine", TETRAHEDRON LETTERS, ELSEVIER, AMSTERDAM , NL, vol. 41, no. 2, 1 January 2000 (2000-01-01), pages 179 - 181, XP004185443, ISSN: 0040-4039, DOI: 10.1016/S0040-4039(99)02049-3**

Remarks:

The file contains technical information submitted after the application was filed and not included in this specification

**Description**

TECHNICAL FIELD

The present invention relates to a process for the production of 2,2,4,6,6-pentamethyl-1,2,5,6-tetrahydro-pyrimidine.

INTRODUCTION

2,2,4,6,6-pentamethyl-1,2,5,6-tetrahydro-pyrimidine is a valuable intermediate in industrial organic chemistry. The preparation of 2,2,4,6,6-pentamethyl-1,2,5,6-tetrahydro-pyrimidine usually proceeds via a complex system of equilibria in a continuous or semi-continuous process in which acetone is converted with ammonia in the presence of a catalyst. Typically, ammonium salts are used as homogeneous catalyst. The type of the conversion may be classified as cyclocondensation reaction, whereby water is set free. In the processes known in the art, however, 2,2,4,6,6-pentamethyl-1,2,5,6-tetrahydro-pyrimidine reacts further to 2,2,6,6-tetramethyl-4-piperidone.

Cavani et al. in Journal of Molecular Catalysis A: Chemical 393 (2014), 325-332, discloses a study on the acid-catalyzed condensation of acetone and ammonia to directly produce 2,2,6,6-tetramethyl-4-piperidineone under both homogeneous and heterogeneous conditions, wherein aqueous ammonia is used as the ammonia source. As catalysts, H-Y zeolites have been tested having selected features. 2,2,4,6,6-pentamethyl-1,2,5,6-tetrahydro-pyrimidine is, however, only formed in lesser amounts as a by-product during the preparation of 2,2,6,6-tetramethyl-4-piperidone. According to Cavani et al. 1,2,5,6-tetrahydro-2,2,4,6,6-pentamethyl-pyrimidine may transform into 2,2,6,6-tetramethyl-4-piperidone either with the insertion of water and elimination of ammonia or, in the presence of acetone, with acetone, with acetone insertion and elimination of 2-iminopropane. Cavani, however, indicates that when using lower reaction temperatures, the selectivity to 1,2,5,6-tetrahydro-2,2,4,6,6-pentamethyl-pyrimidine increased, and at the same time the selectivity to 2,2,6,6-tetramethyl-4-piperidone decreased.

DE 1 276 041 B, on the other hand, relates to the production of 2,2,4,6,6-pentamethyl-1,2,5,6-tetrahydro-pyrimidine by reaction of acetone and ammonia over montmorillonite-like minerals as an acid catalyst.

In general, however, there remains the need to develop an improved process for the production of 2,2,4,6,6-pentamethyl-1,2,5,6-tetrahydro-pyrimidine where the formation of by-products is avoided or at least diminished.

DETAILED DESCRIPTION

Therefore, it was an object of the present invention to provide an improved process for the production of 2,2,4,6,6-pentamethyl-1,2,5,6-tetrahydro-pyrimidine, in particular as regards an improved selectivity towards 2,2,4,6,6-pentamethyl-1,2,5,6-tetrahydro-pyrimidine. In particular, it was an object to entirely avoid or at least diminish the formation of specific by-products, in particular of 2,2,6,6-tetramethyl-4-piperidone.

Thus, it has surprisingly been found that use of a catalyst comprising a zeolitic material having specific acidity characteristics in a heterogeneous process for production of 2,2,4,6,6-pentamethyl-1,2,5,6-tetrahydro-pyrimidine leads to an improved selectivity towards 2,2,4,6,6-pentamethyl-1,2,5,6-tetrahydro-pyrimidine, wherein in particular the formation of 2,2,6,6-tetramethyl-4-piperidone is reduced, if not entirely suppressed.

Therefore, the present invention relates to a process for the production of 2,2,4,6,6-pentamethyl-1,2,5,6-tetrahydro-pyrimidine comprising

(i) providing a reactor containing a catalyst comprising a zeolitic material, wherein the zeolitic material comprises $YO_2$ and optionally comprises $X_2O_3$ in its framework structure, wherein Y is a tetravalent element and X is a trivalent element;

(ii) preparing a reaction mixture comprising acetone and ammonia;

(iii) contacting the catalyst in the reactor with the reaction mixture prepared in (ii) for obtaining a reaction product comprising 2,2,4,6,6-pentamethyl-1,2,5,6-tetrahydro-pyrimidine;

wherein the temperature programmed desorption of ammonia ($NH_3$-TPD) profile of the zeolitic material comprised in the catalyst provided in (i), displays one or more bands associated with medium acid sites, said one or more bands having maxima in the temperature range of from 250 to 500°C, wherein the integration of said one or more bands affords a total value of 0.5 mmol/g or less, wherein the $NH_3$-TPD profile is preferably determined according to Reference Example 2, and wherein the mixture prepared in (ii) and contacted with the catalyst in (iii) contains water in an amount in the range of from 0.01 to 10 wt.-%, preferably in the range of from 0.01 to 6 wt.-%, more preferably in the range of from 0.05 to 4 wt.-%, more preferably in the range of from 0.1 to 1 wt.-%, more preferably in the range of from 0.15 to 0.5 wt.-%, more preferably in the range of from 0.2 to 0.3 wt.-%, based on 100 wt.-% of the reaction mixture.

Preferably, the $NH_3$-TPD profile of the zeolitic material comprised in the catalyst provided in (i), preferably wherein the $NH_3$-TPD profile of the catalyst provided in (i), displays one or more bands associated with medium acid sites, said one or more bands having maxima in the temperature range of from 250 to 500°C, wherein the integration of said one or more bands affords a total value of 0.45 mmol/g or less, preferably of 0.4 mmol/g or less, more preferably of 0.35 mmol/g or less, more preferably of 0.3 mmol/g or less, more preferably of 0.25 mmol/g or less, more preferably of 0.2 mmol/g or less, more preferably of 0.15 mmol/g or less, more preferably of 0.1 mmol/g or less, more preferably of 0.05 mmol/g or less, more preferably of 0.03 mmol/g or less, more preferably of 0.02 mmol/g or less, more preferably of 0.015 mmol/g or less, more preferably of 0.01 mmol/g or less, more preferably of 0.005 mmol/g or less, and more preferably of 0.001 mmol/g or less, wherein the $NH_3$-TPD profile is preferably determined according to Reference Example 2.

Preferably, the one or more bands associated with medium acid sites have their maxima in the temperature range of from 260 to 480°C, preferably of from 270 to 450°C, more preferably of from 275 to 430°C, more preferably of from 280 to 400°C, more preferably of from 285 to 380°C, more preferably of from 290 to 350°C, more preferably of from 295 to 330°C, and more preferably of from 300 to 310°C.

Preferably, the $NH_3$-TPD profile of the zeolitic material comprised in the catalyst provided in (i) displays one or more bands associated with strong acid sites, said one or more bands having maxima in the temperature range of from 510 to 800°C, wherein the integration of said one or more bands affords a total value of 0.05 mmol/g or less, preferably of 0.04 mmol/g or less, more preferably of 0.03 mmol/g or less, more preferably of 0.02 mmol/g or less, more preferably of 0.015 mmol/g or less, more preferably of 0.01 mmol/g or less, more preferably of 0.005 mmol/g or less, and more preferably of 0.001 mmol/g or less, wherein the $NH_3$-TPD profile is preferably determined according to Reference Example 2.

It is further preferred that the $NH_3$-TPD profile of the zeolitic material comprised in the catalyst provided in (i) displays one or more bands associated with strong acid sites, said one or more bands having maxima in the temperature range of from 510 to 800°C, wherein the integration of said one or more bands affords a total value in the range of from 0.001 to 0.070 mmol/g, preferably in the range of from 0.001 to 0.062 mmol/g, more preferably in the range of from 0.002 to 0.024 mmol/g, more preferably in the range of from 0.003 to 0.013 mmol/g, more preferably in the range 0.004 to 0.006 mmol/g, wherein the $NH_3$-TPD profile is preferably determined according to Reference Example 2.

Further preferably, the one or more bands associated with strong acid sites have their maxima in the temperature range of from 530 to 750°C, preferably of from 550 to 700°C, more preferably of from 560 to 650°C, more preferably of from 570 to 630°C, more preferably of from 575 to 620°C, more preferably of from 580 to 610°C, more preferably of from 585 to 605°C, and more preferably of from 590 to 600°C.

Preferably, the integration of the bands in the $NH_3$-TPD profile of the zeolitic material comprised in the catalyst provided in (i), said bands having maxima in the temperature range of from 50 to 800°C, affords a total value of 0.9 mmol/g or less, preferably of 0.7 mmol/g or less, more preferably of 0.5 mmol/g or less, more preferably of 0.3 mmol/g or less, more preferably of 0.2 mmol/g or less, more preferably of 0.15 mmol/g or less, more preferably of 0.1 mmol/g or less, more preferably of 0.08 mmol/g or less, more preferably of 0.06 mmol/g or less, more preferably of 0.04 mmol/g or less, more preferably of 0.03 mmol/g or less, more preferably of 0.02 mmol/g or less, more preferably of 0.01 mmol/g or less, and more preferably of 0.005 mmol/g or less, wherein the $NH_3$-TPD profile is preferably determined according to Reference Example 2. Further preferably, the bands have their maxima in the temperature range of from 80 to 750°C, preferably of from 100 to 700°C, more preferably of from 130 to 650°C, more preferably of from 150 to 600°C, more preferably of from 180 to 580°C, more preferably of from 200 to 550°C, and more preferably of from 230 to 530°C.

Preferably, the $NH_3$-TPD profile is a deconvoluted $NH_3$-TPD profile, wherein the one or more bands of the $NH_3$-TPD profile are the one or more deconvoluted bands of the $NH_3$-TPD profile, wherein deconvolution of the bands is preferably achieved according to Reference Example 2.

Preferably, the zeolitic material has a framework structure having a maximum ring size of 10 T-atoms or less, wherein the zeolitic material preferably has a framework structure type selected from the group consisting of MFI, FER, HEU, MEL, MWW, RRO, and TON, including mixed structures of two or more thereof, preferably from the group consisting of MFI, FER, and MWW, including mixed structures of two or more thereof, wherein more preferably the zeolitic material has an MFI and/or FER-type framework structure, wherein more preferably the zeolitic material has an MFI-type framework structure.

Preferably, from 5 weight-% or more of the catalyst consist of the zeolitic material, preferably 10 weight-% or more, more preferably 30 weight-% or more, more preferably 50 weight-% or more, more preferably 70 weight-% or more, more preferably 80 weight-% or more, more preferably 90 weight-% or more, more preferably 95 to 100 weight-%, preferably from 99 to 100 weight-%, and more preferably from 99.5 to 100 weight-% of the catalyst consist of the zeolitic material.

Preferably, the catalyst is provided as a powder and/or as a molding, preferably as an extrudate.

According to the present invention, a molding is to be understood as a three-dimensional entity obtained from a shaping process; accordingly, the term "molding" is used synonymously with the term "shaped body".

Preferably, Y is selected from the group consisting of Si, Sn, Ti, Zr, Ge, and mixtures of two or more thereof, Y preferably being Si.

Alternatively, it is preferred that Y comprises Si and Sn, or Si and Ge, or Si and Zr, more preferably Si and Sn, wherein Y more preferably is Si and Sn, or Si and Ge, or Si and Zr, more preferably Si and Sn.

Alternatively, it is preferred that Y comprises Si and Ti, wherein more preferably Y is Si and Ti.

Preferably, X is selected from the group consisting of B, Al, Ga, In, and mixtures of two or more thereof, wherein preferably X is Al and/or B, wherein more preferably X is Al.

Preferably, the zeolitic material provided in (i) contains one or more transition metal elements, wherein the catalyst provided in (i) is obtained and/or obtainable by a process comprising loading one or more salts of the one or more transition metal elements into the pores of the porous structure of the zeolitic material and optionally on the surface of the zeolitic material. Further preferably, the one or more transition metal elements are selected from the group consisting of Sc, Y, La, Hf, Ce, Dy, Er, Eu, Gd, Ho, La, Lu, Nd, Pr, Pm, Sm, Sc, Tb, Tm, Yb, Sn, Fe, Co, Ni, and combinations of two or more thereof, preferably from the group consisting of Sc, Y, La, Hf, Ce, Sn, Fe, Co, Ni, and combinations of two or more thereof, more preferably from the group consisting of Sc, La, Hf, Ce, Sn, Fe, and combinations of two or more thereof, and more preferably from the group consisting of Sc, La, Hf, Ce, Sn, and combinations of two or more thereof, wherein more preferably the one or more transition metals comprise La and/or Sn, preferably La, wherein more preferably the one or more transition metals are La and/or Sn, preferably La.

Further preferably, the zeolitic material contains 0.1 to 15 weight-% of the one or more transition metal elements, calculated as the element and based on 100 weight-% of $YO_2$ contained in the framework structure of the zeolitic material, preferably from 0.5 to 12 weight-%, more preferably from 1 to 10 weight-%, more preferably from 2 to 9 weight-%, more preferably from 3 to 8 weight-%, more preferably from 3.5 to 7 weight-%, more preferably from 4 to 6 weight-%, and more preferably from 4.5 to 5.5 weight-%.

Further preferably, the loading of the one or more salts of the one or more transition metal elements into the pores of the porous structure of the zeolitic material and optionally on the surface of the zeolitic material comprises

(a) impregnating the porous structure of the zeolitic material with a solution of the one or more salts of the one or more transition metal elements;
(b) optionally drying the impregnated zeolitic material obtained in (b);
(c) calcining the zeolitic material obtained in (a) or (b).

Yet further preferably, the solution in (a) is an aqueous solution, wherein preferably the solution consists of the one or more salts of the one or more transition metal elements dissolved in distilled water.

Yet further preferably, the volume of the solution employed in (a) is equal to 500% or less of the total pore volume of the zeolitic material prior to impregnation with the solution, wherein preferably the volume of the solution employed in (a) is equal to 50 to 350% of the total pore volume of the zeolitic material prior to impregnation with the solution, more preferably to 100 to 300%, more preferably to 150 to 270%, more preferably to 180 to 250%, more preferably to 200 to 230%, and more preferably to 210 to 220%, wherein the total pore volume is determined by nitrogen adsorption from the BJH method, preferably according to DIN 66134.

Yet further preferably, (a) is conducted at a temperature in the range of from 5 to 40 °C, preferably from 10 to 35 °C, more preferably from 15 to 30 °C, and more preferably from 20 to 25 °C.

Yet further preferably, the one or more salts are selected from the group consisting of halides, preferably chloride and/or bromide, more preferably chloride, hydroxide, sulfate, nitrate, phosphate, acetate, and mixtures of two or more thereof, more preferably from the group consisting of chloride, acetate, nitrate, and mixtures of two or more thereof, wherein more preferably the one or more salts are nitrates.

Yet further preferably, the loading of the one or more salts of the one or more transition metal elements into the pores of the porous structure of the zeolitic material and optionally on the surface of the zeolitic material comprises

(a') preparing a mixture of the one or more salts of the one or more transition metal elements and the zeolitic material;
(b') optionally milling the mixture obtained in (a');
(c') calcining the zeolitic material obtained in (a') or (b').

Yet further preferably, the one or more salts in (a') are selected from the group consisting of halides, preferably chloride and/or bromide, more preferably chloride, hydroxide, sulfate, nitrate, phosphate, acetate, and mixtures of two or more thereof, more preferably from the group consisting of chloride, acetate, nitrate, and mixtures of two or more thereof, wherein more preferably the one or more salts are nitrates.

Yet further preferably, calcining in (c) or (c') is conducted at a temperature in the range of from 300 to 900 °C, preferably of from 350 to 700 °C, more preferably of from 400 to 600 °C, and more preferably of from 450 to 550 °C. Preferably, calcining in (c) or (c') is conducted in air.

Yet further preferably, prior to the loading of the one or more salts of the one or more transition metal elements into the pores of the porous structure of the zeolitic material and optionally on the surface of the zeolitic material in (a) or (a'), the zeolitic material is in the H-form and contains protons as extra-framework ions, wherein 0.1 weight-% or less of the extra-framework ions are metal cations, calculated as the element and based on 100 weight-% of $YO_2$ contained in the zeolitic material, preferably 0.05 weight-% or less, more preferably 0.001 weight-% or less, more preferably 0.0005 weight-% or less, and more preferably 0.0001 weight-% or less.

Preferably, the zeolitic material contains substantially no Na, preferably substantially no Na or K, more preferably substantially no alkali metal, and more preferably substantially no alkali metal or alkaline earth metal.

Preferably, the zeolitic material has a $YO_2 : X_2O_3$ molar ratio in the range of from 5 to 300, preferably from 10 to 200, more preferably from 15 to 150, more preferably from 20 to 120, more preferably from 25 to 100, more preferably from 30 to 80, more preferably from 35 to 70, more preferably from 40 to 60, and more preferably from 45 to 55.

Preferably, the zeolitic material provided in (i) comprises a zeolitic material having an MFI framework-type structure, wherein the zeolitic material preferably comprises one or more zeolites selected from the group consisting of Silicalite , ZSM-5, [Fe-Si-O]-MFI, Monoclinic H-ZSM-5, [Ga-Si-O]-MFI, [As-Si-O]-MFI, AMS-1B, AZ-1, Bor-C, Encilite, Boralite C, FZ-1, LZ-105, Mutinaite, NU-4, NU-5, TS-1, TSZ, TSZ-III, TZ-01, USC-4, USI-108, ZBH, ZKQ-1B, ZMQ-TB, organic-free ZSM-5, and mixtures of two or more thereof, more preferably from the group consisting of ZSM-5, AMS-1B, AZ-1, FZ-1, LZ-105, NU-4, NU-5, TS-1, TSZ, TSZ-III, TZ-01, USC-4, USI-108, ZBH, ZKQ-1B, ZMQ-TB, and mixtures of two or more thereof, wherein more preferably the zeolitic material comprises TS-1 and/or ZSM-5, wherein more preferably the zeolitic material comprises TS-1, wherein more preferably the zeolitic material is TS-1 and/or ZSM-5, wherein more preferably the zeolitic material is TS-1.

Preferably, the zeolitic material provided in (i) comprises a zeolitic material having an FER framework-type structure, wherein the zeolitic material preferably comprises one or more zeolites selected from the group consisting of ferrierite, ZSM-35, NU-23, FU-23, ISI-6, [Si-O]-FER, [Ga-Si-O]-FER, [B-Si-O]-FER, and mixtures of two or more thereof, more preferably from the group consisting of ferrierite, ZSM-35, NU-23, FU-23, ISI-6, [Si-O]-FER, and mixtures of two or more thereof, wherein more preferably the zeolitic material comprises ferrierite, wherein more preferably the zeolitic material is ferrierite.

No particular restriction applies as regards the chemical or physical nature of the zeolitic material. Preferably, the zeolitic material has a crystallinity in the range of from 50 to 100 weight-%, preferably from 75 to 100 weight-%, more preferably from 80 to 100 weight-%, and more preferably from 90 to 100 weight-%, wherein the crystallinity is preferably determined as described in Reference Example 3.

Preferably, the zeolitic material has a BET specific surface area in the range of from 100 to 600 $m^2/g$, preferably from 150 to 500 $m^2/g$, more preferably from 200 to 450 $m^2/g$, more preferably from 225 to 425 $m^2/g$, more preferably from 250 to 400 $m^2/g$, more preferably from 275 to 375 $m^2/g$, more preferably from 300 to 350 $m^2/g$, and more preferably from 315 to 335 $m^2/g$, wherein the BET surface area is preferably determined according to ISO 9277:2010, and is more preferably determined as described in Reference Example 1.

Preferably, the zeolitic material provided in (i) is a calcined zeolitic material, wherein the zeolitic material has been calcined at a temperature in the range of from 300 to 900°C, preferably of from 400 to 700°C, more preferably of from 450 to 650°C, and more preferably of from 500 to 600°C.

Preferably, the zeolitic material has been calcined for a duration of from 0.5 to 24 h, preferably from 1 to 15 h, more preferably from 2 to 12 h, more preferably from 2.5 to 9 h, more preferably from 3 to 7 h, more preferably from 3.5 to 6.5 h, and more preferably from 4 to 6 h.

Preferably, prior to contacting of the catalyst with the reaction mixture in (ii) the catalyst is first contacted with acetone.

Preferably, the process is conducted as a batch process or as a continuous process, wherein preferably the process is conducted as a continuous process.

Preferably, the process is conducted as a batch process, wherein in (iii) the contacting of the catalyst with the reaction mixture prepared in (ii) is conducted at a temperature in the range of from 35 to 250 °C, preferably from 40 to 200 °C, more preferably from 45 to 150 °C, more preferably from 50 to 100 °C, more preferably in the range of from 55 to 80 °C, and more preferably in the range of from 60 to 70 °C.

Preferably, the process is conducted as a batch process, wherein in (iii) the contacting of the catalyst with the reaction mixture prepared in (ii) is conducted at a pressure in the range of from 1 to 30 bar(abs), preferably in the range of from 1 to 20 bar(abs), more preferably in the range of from 1 to 15 bar(abs), more preferably in the range of from 1 to 10 bar(abs), more preferably in the range of from 1 to 5, more preferably in the range of from 1 to 3 bar(abs).

Preferably, the process is conducted as a batch process, wherein contacting in (iii) is carried out for a duration in the range of from 0.1 to 24 h, preferably in the range of from 0.5 to 20 h, more preferably in the range of from 1 to 15 h, more preferably in the range of from 3 to 10 h, more preferably in the range of from 4 to 8 h, more preferably in the range of from 5 to 7 h.

Alternatively, the process is preferably conducted as a continuous process, wherein in (iii) the contacting of the catalyst with the reaction mixture prepared in (ii) is conducted at a pressure in the range of from 1 to 200 bar(abs), preferably in the range of from 20 to 150 bar(abs), more preferably in the range of from 30 to 100 bar(abs), more preferably in the range of from 40 to 80 bar(abs), more preferably in the range of from 45 to 70, more preferably in the range of from 50 to 60 bar(abs).

The unit bar(abs) refers to an absolute pressure of $10^5$ Pa and the unit Angstrom refers to a length of $10^{-10}$ m.

Preferably, the process is conducted as a continuous process, wherein contacting in (iii) is conducted at a liquid hourly space velocity (LHSV) in the range of from 0.01 to 1000 ml/(g·h), preferably from 0.05 to 500 ml/(g·h), more preferably from 0.1 to 100 ml/(g·h), more preferably from 0.3 to 10 ml/(g·h), more preferably from 0.5 to 5 ml/(g·h), more preferably from 1 to 4 ml/(g·h), more preferably from 1.2 to 3 ml/(g·h), more preferably from 1.5 to 2 ml/(g·h).

Preferably, the process is conducted as a continuous process, wherein in (iii) the contacting of the catalyst with the reaction mixture prepared in (ii) is conducted at a temperature in the range of from 0 to 150 °C, preferably from 5 to 100 °C, more preferably from 10 to 50 °C, more preferably from 15 to 40 °C, more preferably in the range of from 20 to 35 °C, and more preferably in the range of from 25 to 30 °C.

Preferably, the reaction product obtained in (iii) further comprises water, wherein the reaction product obtained in (iii) more preferably contains water in an amount in the range of from 0.01 to 10 wt.-%, preferably in the range of from 1 to 8 wt.-%, more preferably in the range of from 2 to 6 wt.-%, more preferably in the range of from 2.5 to 4.5 wt.-%, more preferably in the range of from 2.9 to 3.8 wt.-%, more preferably in the range of from 3.0 to 3.7 wt.-%, based on 100 wt.-% of the reaction product.

Preferably, the process further comprises

(iv) separating 2,2,4,6,6-pentamethyl-1,2,5,6-tetrahydro-pyrimidine from the reaction product to obtain 2,2,4,6,6-pentamethyl-1,2,5,6-tetrahydro-pyrimidine and a residual mixture;
(v) recycling at least a portion of the residual mixture in the reaction mixture in (ii),
wherein preferably after (iv) and prior to (v) compounds having a boiling point higher than that of 2,2,4,6,6-pentamethyl-1,2,5,6-tetrahydro-pyrimidine were separated from the residual mixture.

Preferably, in the reaction mixture prepared in (ii), the molar ratio of acetone to ammonia is in the range of from 1:1 to 25:1, preferably of from 2:1 to 22:1, more preferably of from 4:1 to 20:1, more preferably of from 6:1 to 18:1, more preferably of from 8:1 to 17:1, more preferably of from 10:1 to 16:1, more preferably in the range of from 12:1.0 to 14.7:1.0, more preferably in the range of from 14.2:1.0 to 14.5:1.0.

Preferably, the catalyst is provided as a molding, wherein the molding is prepared according to a process comprising

(a) preparing a mixture comprising the zeolitic material and optionally one or more binders;
(b) shaping the mixture, preferably by extruding to obtain a shaped material;
(c) optionally drying the shaped material in a gas atmosphere;
(d) calcining the shaped material obtained from (b) or (c) in a gas atmosphere to obtain the molding.

Further preferably, the mixture in (a) comprises one or more binders, wherein the one or more binders is one or more of alumina, silica, silica-alumina, graphite, a polysaccharide, a sugar alcohol and a synthetic polymer, preferably one or more of alumina, silica, silica-alumina, graphite, a sugar alcohol, a synthetic polymer, cellulose, a modified cellulose and a starch, more preferably silica, alumina, silica-alumina, graphite, a sugar alcohol, a synthetic polymer, a microcrystalline cellulose, a cellulose ether, more preferably graphite, sorbitol, mannitol, polyethylene glycol (PEG), polyvinylpyrrolidone (PVP), hydroxypropyl cellulose (HPC), hydroxypropyl methylcellulose (HPMC), and sodium carboxymethyl cellulose.

Further preferably, in the mixture according to (a), the weight ratio of the one or more binders relative to the zeolitic material is in the range of from 1:10 to 1:30, preferably in the range of from 1:15 to 1:25.

Further preferably, the process comprises drying according to (c), wherein drying is conducted in a gas atmosphere having a temperature in the range of from 90 to 150 °C, preferably at a temperature in the range of from 110 to 130 °C, wherein preferably the drying is conducted for 10 to 15 h, more preferably for 11 to 13 h.

Further preferably, the gas atmosphere in (d) has a temperature in the range of from 400 to 600 °C, preferably in the range of from 450 to 550 °C, wherein preferably the calcining is conducted for 3 to 7 h, more preferably for 4 to 6 h.

Further preferably, the gas atmosphere in one or more of (c) and (d) comprises one or more of nitrogen, oxygen, and argon, preferably nitrogen, wherein preferably the gas atmosphere in one or more of (c) and (d) comprises air, wherein more preferably the gas atmosphere in one or more of (c) and (d) consists of air.

The present invention is further illustrated by the following set of embodiments and combinations of embodiments resulting from the dependencies and back-references as indicated. In particular, it is noted that in each instance where a range of embodiments is mentioned, for example in the context of a term such as "any one of embodiments (1) to (4)", every embodiment in this range is meant to be explicitly disclosed for the skilled person, i.e. the wording of this term is to be understood by the skilled person as being synonymous to "any one of embodiments (1), (2), (3), and (4)". Further, it is explicitly noted that the following set of embodiments is not the set of claims determining the extent of protection, but represents a suitably structured part of the description directed to general and preferred aspects of the present invention.

According to an embodiment (1), the present invention relates to a process for the production of 2,2,4,6,6-pentamethyl-1,2,5,6-tetrahydro-pyrimidine comprising

(i) providing a reactor containing a catalyst comprising a zeolitic material, wherein the zeolitic material comprises $YO_2$ and optionally comprises $X_2O_3$ in its framework structure, wherein Y is a tetravalent element and X is a trivalent element;
(ii) preparing a reaction mixture comprising acetone and ammonia;
(iii) contacting the catalyst in the reactor with the reaction mixture prepared in (ii) for obtaining a reaction product comprising 2,2,4,6,6-pentamethyl-1,2,5,6-tetrahydro-pyrimidine;

wherein the temperature programmed desorption of ammonia ($NH_3$-TPD) profile of the zeolitic material comprised in the catalyst provided in (i), preferably wherein the $NH_3$-TPD profile of the catalyst provided in (i), displays one or more bands associated with medium acid sites, said one or more bands having maxima in the temperature range of from 250 to 500°C, wherein the integration of said one or more bands affords a total value of 0.5 mmol/g or less, wherein the $NH_3$-TPD profile is preferably determined according to Reference Example 2, and wherein the mixture prepared in (ii) and contacted with the catalyst in (iii) contains water in an amount in the range of from 0.01 to 10wt.-%, more preferably in the range of from 0.01 to 6 wt-%, more preferably in the range of from 0.05 to 4 wt.-%, more preferably in the range of from 0.1 to 1 wt.-%, more preferably in the range of from 0.15 to 0.5 wt.-%, more preferably in the range of from 0.2 to 0.3 wt.-%, based on 100 wt.-% of the reaction mixture.

A preferred embodiment (2) concretizing embodiment (1) wherein the $NH_3$-TPD profile of the zeolitic material comprised in the catalyst provided in (i), preferably wherein the $NH_3$-TPD profile of the catalyst provided in (i), displays one or more bands associated with medium acid sites, said one or more bands having maxima in the temperature range of from 250 to 500°C, wherein the integration of said one or more bands affords a total value of 0.45 mmol/g or less, preferably of 0.4 mmol/g or less, more preferably of 0.35 mmol/g or less, more preferably of 0.3 mmol/g or less, more preferably of 0.25 mmol/g or less, more preferably of 0.2 mmol/g or less, more preferably of 0.15 mmol/g or less, more preferably of 0.1 mmol/g or less, more preferably of 0.05 mmol/g or less, more preferably of 0.03 mmol/g or less, more preferably of 0.02 mmol/g or less, more preferably of 0.015 mmol/g or less, more preferably of 0.01 mmol/g or less, more preferably of 0.005 mmol/g or less, and more preferably of 0.001 mmol/g or less, wherein the $NH_3$-TPD profile is preferably determined according to Reference Example 2.

A preferred embodiment (3) concretizing any one of embodiments (1) to (2), wherein the one or more bands associated with medium acid sites have their maxima in the temperature range of from 260 to 480°C, preferably of from 270 to 450°C, more preferably of from 275 to 430°C, more preferably of from 280 to 400°C, more preferably of from 285 to 380°C, more preferably of from 290 to 350°C, more preferably of from 295 to 330°C, and more preferably of from 300 to 310°C.

A preferred embodiment (4) concretizing any one of embodiments (1) to (3), wherein the $NH_3$-TPD profile of the zeolitic material comprised in the catalyst provided in (i) displays one or more bands associated with strong acid sites, said one or more bands having maxima in the temperature range of from 510 to 800°C, wherein the integration of said one or more bands affords a total value of 0.05 mmol/g or less, preferably of 0.04 mmol/g or less, more preferably of 0.03 mmol/g or less, more preferably of 0.02 mmol/g or less, more preferably of 0.015 mmol/g or less, more preferably of 0.01 mmol/g or less, more preferably of 0.005 mmol/g or less, and more preferably of 0.001 mmol/g or less, wherein the $NH_3$-TPD profile is preferably determined according to Reference Example 2.

A preferred embodiment (5) concretizing any one of embodiments (1) to (4), wherein the $NH_3$-TPD profile of the zeolitic material comprised in the catalyst provided in (i) displays one or more bands associated with strong acid sites, said one or more bands having maxima in the temperature range of from 510 to 800°C, wherein the integration of said one or more bands affords a total value in the range of from 0.001 to 0.070 mmol/g, preferably in the range of from 0.001 to 0.062 mmol/g, more preferably in the range of from 0.002 to 0.024 mmol/g, more preferably in the range of from 0.003 to 0.013 mmol/g, more preferably in the range 0.004 to 0.006 mmol/g, wherein the $NH_3$-TPD profile is preferably determined according to Reference Example 2.

A preferred embodiment (6) concretizing embodiment (4) or (5), wherein the one or more bands associated with strong acid sites have their maxima in the temperature range of from 530 to 750°C, preferably of from 550 to 700°C, more preferably of from 560 to 650°C, more preferably of from 570 to 630°C, more preferably of from 575 to 620°C, more preferably of from 580 to 610°C, more preferably of from 585 to 605°C, and more preferably of from 590 to 600°C.

A preferred embodiment (7) concretizing any one of embodiments (1) to (6), wherein the integration of the

bands in the NH$_3$-TPD profile of the zeolitic material comprised in the catalyst provided in (i), said bands having maxima in the temperature range of from 50 to 800°C, affords a total value of 0.9 mmol/g or less, preferably of 0.7 mmol/g or less, more preferably of 0.5 mmol/g or less, more preferably of 0.3 mmol/g or less, more preferably of 0.2 mmol/g or less, more preferably of 0.15 mmol/g or less, more preferably of 0.1 mmol/g or less, more preferably of 0.08 mmol/g or less, more preferably of 0.06 mmol/g or less, more preferably of 0.04 mmol/g or less, more preferably of 0.03 mmol/g or less, more preferably of 0.02 mmol/g or less, more preferably of 0.01 mmol/g or less, and more preferably of 0.005 mmol/g or less, wherein the NH$_3$-TPD profile is preferably determined according to Reference Example 2.

A preferred embodiment (8) concretizing embodiment (7), wherein the bands have their maxima in the temperature range of from 80 to 750°C, preferably of from 100 to 700°C, more preferably of from 130 to 650°C, more preferably of from 150 to 600°C, more preferably of from 180 to 580°C, more preferably of from 200 to 550°C, and more preferably of from 230 to 530°C.

A preferred embodiment (9) concretizing any one of embodiments (1) to (8), wherein the NH$_3$-TPD profile is a deconvoluted NH$_3$-TPD profile, and wherein the one or more bands of the NH$_3$-TPD profile are the one or more deconvoluted bands of the NH$_3$-TPD profile, wherein deconvolution of the bands is preferably achieved according to Reference Example 2.

A preferred embodiment (10) concretizing any one of embodiments (1) to (9), wherein the zeolitic material has a framework structure having a maximum ring size of 10 T-atoms or less, wherein the zeolitic material preferably has a framework structure type selected from the group consisting of MFI, FER, HEU, MEL, MWW, RRO, and TON, including mixed structures of two or more thereof, preferably from the group consisting of MFI, FER, and MWW, including mixed structures of two or more thereof, wherein more preferably the zeolitic material has an MFI and/or FER-type framework structure, wherein more preferably the zeolitic material has an MFI-type framework structure.

A preferred embodiment (11) concretizing any one of embodiments (1) to (10), wherein from 5 weight-% or more of the catalyst consist of the zeolitic material, preferably 10 weight-% or more, more preferably 30 weight-% or more, more preferably 50 weight-% or more, more preferably 70 weight-% or more, more preferably 80 weight-% or more, more preferably 90 weight-% or more, more preferably 95 to 100 weight-%, preferably from 99 to 100 weight-%, and more preferably from 99.5 to 100 weight-% of the catalyst consist of the zeolitic material.

A preferred embodiment (12) concretizing any one of embodiments (1) to (11), wherein the catalyst is provided as a powder and/or as a molding, preferably as an extrudate.

A preferred embodiment (13) concretizing any one of embodiments (1) to (12), wherein Y is selected from the group consisting of Si, Sn, Ti, Zr, Ge, and mixtures of two or more thereof, Y preferably being Si.

A preferred embodiment (14) concretizing any one of embodiments (1) to (13), wherein Y comprises, preferably consists of, Si and Sn, or Si and Ge, or Si and Zr, wherein Y preferably is Si and Sn.

A preferred embodiment (15) concretizing any one of embodiments (1) to (13), wherein Y comprises Si and Ti, wherein more preferably Y is Si and Ti.

A preferred embodiment (16) concretizing any one of embodiments (1) to (15), wherein X is selected from the group consisting of B, Al, Ga, In, and mixtures of two or more thereof, wherein preferably X is Al and/or B, wherein more preferably X is Al.

A preferred embodiment (17) concretizing any one of embodiments (1) to (16), wherein the zeolitic material provided in (i) contains one or more transition metal elements, wherein the catalyst provided in (i) is obtained and/or obtainable by a process comprising loading one or more salts of the one or more transition metal elements into the pores of the porous structure of the zeolitic material and optionally on the surface of the zeolitic material.

A preferred embodiment (18) concretizing embodiment (17), wherein the one or more transition metal elements are selected from the group consisting of Sc, Y, La, Hf, Ce, Dy, Er, Eu, Gd, Ho, La. Lu. Nd. Pr. Pm. Sm. Sc. Tb. Tm. Yb. Sn. Fe. Co. Ni, and combinations of two or more thereof, preferably from the group consisting of Sc, Y, La, Hf, Ce, Sn, Fe, Co, Ni, and combinations of two or more thereof, more preferably from the group consisting of Sc, La, Hf, Ce, Sn, Fe, and combinations of two or more thereof, and more preferably from the group consisting of Sc, La, Hf, Ce, Sn, and combinations of two or more thereof, wherein more preferably the one or more transition metals comprise La and/or Sn, preferably La, wherein more preferably the one or more transition metals are La and/or Sn, preferably La.

A preferred embodiment (19) concretizing embodiment (17) or (18), wherein the zeolitic material contains 0.1 to 15 weight-% of the one or more transition metal elements, calculated as the element and based on 100 weight-% of YO$_2$ contained in the framework structure of the zeolitic material, preferably from 0.5 to 12 weight-%, more preferably from 1 to 10 weight-%, more preferably from 2 to 9 weight-%, more preferably from 3 to 8 weight-%, more preferably from 3.5 to 7 weight-%, more preferably from 4 to 6 weight-%, and more preferably from 4.5 to 5.5 weight-%.

A preferred embodiment (20) concretizing any one of embodiments (17) to (19) wherein the loading of the one or more salts of the one or more transition metal elements into the pores of the porous structure of the zeolitic material and optionally on the surface of the zeolitic material comprises

    (a) impregnating the porous structure of the zeolitic material with a solution of the one or more salts of the one or more transition metal elements;
(b) optionally drying the impregnated zeolitic material obtained in (b);
(c) calcining the zeolitic material obtained in (a) or (b).

A preferred embodiment (21) concretizing embodiment (20), wherein the solution is an aqueous solution, wherein preferably the solution consists of the one or more salts of the one or more transition metal elements dissolved in distilled water.

A preferred embodiment (22) concretizing embodiment (20) or (21), wherein the volume of the solution employed in (a) is equal to 500% or less of the total pore volume of the zeolitic material prior to impregnation with the solution, wherein preferably the volume of the solution employed in (a) is equal to 50 to 350% of the total pore volume of the zeolitic material prior to impregnation with the solution, more preferably to 100 to 300%, more preferably to 150 to 270%, more preferably to 180 to 250%, more preferably to 200 to 230%, and more preferably to 210 to 220%, wherein the total pore volume is determined by nitrogen adsorption from the BJH method, preferably according to DIN 66134.

A preferred embodiment (23) concretizing any one of embodiments (20) to (22), wherein (a) is conducted at a temperature in the range of from 5 to 40 °C, preferably from 10 to 35 °C, more preferably from 15 to 30 °C, and more preferably from 20 to 25 °C.

A preferred embodiment (24) concretizing any one of embodiments (20) to (23), wherein the one or more salts are selected from the group consisting of halides, preferably chloride and/or bromide, more preferably chloride, hydroxide, sulfate, nitrate, phosphate, acetate, and mixtures of two or more thereof, more preferably from the group consisting of chloride, acetate, nitrate, and mixtures of two or more thereof, wherein more preferably the one or more salts are nitrates.

A preferred embodiment (25) concretizing any one of embodiments (20) to (24), wherein the loading of the one or more salts of the one or more transition metal elements into the pores of the porous structure of the zeolitic material and optionally on the surface of the zeolitic material comprises

(a') preparing a mixture of the one or more salts of the one or more transition metal elements and the zeolitic material;
(b') optionally milling the mixture obtained in (a');
(c') calcining the zeolitic material obtained in (a') or (b').

A preferred embodiment (26) concretizing any one of embodiments (20) to (25), wherein the one or more salts are selected from the group consisting of halides, preferably chloride and/or bromide, more preferably chloride, hydroxide, sulfate, nitrate, phosphate, acetate, and mixtures of two or more thereof, more preferably from the group consisting of chloride, acetate, nitrate, and mixtures of two or more thereof, wherein more preferably the one or more salts are nitrates.

A preferred embodiment (27) concretizing any one of embodiments (20) to (26), wherein calcining in (c) or (c') is conducted at a temperature in the range of from 300 to 900 °C, preferably of from 350 to 700 °C, more preferably of from 400 to 600 °C, and more preferably of from 450 to 550 °C.

A preferred embodiment (28) concretizing any one of embodiments (20) to (27), wherein calcining in (c) or (c') is conducted in air.

A preferred embodiment (29) concretizing any one of embodiments (20) to (28), wherein prior to the loading of the one or more salts of the one or more transition metal elements into the pores of the porous structure of the zeolitic material and optionally on the surface of the zeolitic material in (a) or (a'), the zeolitic material is in the H-form and contains protons as extra-framework ions, wherein 0.1 weight-% or less of the extra-framework ions are metal cations, calculated as the element and based on 100 weight-% of $YO_2$ contained in the zeolitic material, preferably 0.05 weight-% or less, more preferably 0.001 weight-% or less, more preferably 0.0005 weight-% or less, and more preferably 0.0001 weight-% or less.

A preferred embodiment (30) concretizing any one of embodiments (1) to (29), wherein the zeolitic material contains substantially no Na, preferably substantially no Na or K, more preferably substantially no alkali metal, and more preferably substantially no alkali metal or alkaline earth metal.

A preferred embodiment (31) concretizing any one of embodiments (1) to (30), wherein the zeolitic material has a $YO_2 : X_2O_3$ molar ratio in the range of from 5 to 300, preferably from 10 to 200, more preferably from 15 to 150, more preferably from 20 to 120, more preferably from 25 to 100, more preferably from 30 to 80, more preferably from 35 to 70, more preferably from 40 to 60, and more preferably from 45 to 55.

A preferred embodiment (32) concretizing any one of embodiments (1) to (31), wherein the zeolitic material provided in (i) comprises a zeolitic material having an MFI framework-type structure, wherein the zeolitic material preferably comprises one or more zeolites selected from the group consisting of Silicalite , ZSM-5, [Fe-Si-O]-MFI, Monoclinic H-ZSM-5, [Ga-Si-O]-MFI, [As-Si-O]-MFI, AMS-1B, AZ-1, Bor-C, Encilite, Boralite C, FZ-1, LZ-105, Mutinaite, NU-4, NU-5, TS-1, TSZ, TSZ-III, TZ-01, USC-4, USI-108, ZBH, ZKQ-1B, ZMQ-TB, organic-free ZSM-5, and mixtures of two or more thereof, more preferably from the group consisting of ZSM-5, AMS-1B, AZ-1, FZ-1, LZ-105, NU-4, NU-5, TS-1, TSZ, TSZ-III, TZ-01, USC-4, USI-108, ZBH, ZKQ-1B, ZMQ-TB, and mixtures of two or more thereof, wherein more preferably the zeolitic material comprises TS-1 and/or ZSM-5, wherein more preferably the zeolitic material comprises TS-1, wherein more preferably the zeolitic material is TS-1 and/or ZSM-5, wherein more preferably the zeolitic material is TS-1.

A preferred embodiment (33) concretizing any one of embodiments (1) to (32), wherein the zeolitic material provided in (i) comprises a zeolitic material having an FER framework-type structure, wherein the zeolitic material preferably comprises one or more zeolites selected from the group consisting of ferrierite, ZSM-35, NU-23, FU-23, ISI-6, [Si-O]-FER, [Ga-Si-O]-FER, [B-Si-O]-FER, and mixtures of two or more thereof, more preferably from the group consisting of ferrierite, ZSM-35, NU-23, FU-23, ISI-6, [Si-O]-FER, and mixtures of two or more thereof, wherein more preferably the zeolitic material comprises ferrierite, wherein more preferably the zeolitic material is ferrierite.

A preferred embodiment (34) concretizing any one of embodiments (1) to (33), wherein the zeolitic material has a crystallinity in the range of from 50 to 100 weight-%, preferably from 75 to 100 weight-%, more preferably from 80 to 100 weight-%, and more preferably from 90 to 100 weight-%, wherein the crystallinity is preferably determined as described in Reference Example 3.

A preferred embodiment (35) concretizing any one of embodiments (1) to (34), wherein the zeolitic material has a BET specific surface area in the range of from 100 to 600 $m^2/g$, preferably from 150 to 500 $m^2/g$, more preferably from 200 to 450 $m^2/g$, more preferably from 225 to 425 $m^2/g$, more preferably from 250 to 400 $m^2/g$, more preferably from 275 to 375 $m^2/g$, more preferably from 300 to 350 $m^2/g$, and more preferably from 315 to 335 $m^2/g$, wherein the BET surface area is preferably determined according to ISO 9277:2010, and is more preferably determined as described in Reference Example 1.

A preferred embodiment (36) concretizing any one of embodiments (1) to (35), wherein the zeolitic material provided in (i) is a calcined zeolitic material, wherein the zeolitic material has been calcined at a temperature in the range of from 300 to 900°C, preferably of from 400 to 700°C, more preferably of from 450 to 650°C, and more preferably of from 500 to 600°C.

A preferred embodiment (37) concretizing any one of embodiments (1) to (36), wherein the zeolitic material has been calcined for a duration of from 0.5 to 24 h, preferably from 1 to 15 h, more preferably from 2 to 12 h, more preferably from 2.5 to 9 h, more preferably from 3 to 7 h, more preferably from 3.5 to 6.5 h, and more preferably from 4 to 6 h.

A preferred embodiment (38) concretizing any one of embodiments (1) to (37), wherein prior to contacting of the catalyst with the reaction mixture in (ii) the catalyst is first contacted with acetone.

A preferred embodiment (39) concretizing any one of embodiments (1) to (38), wherein the process is conducted as a batch process or as a continuous process, wherein preferably the process is conducted as a continuous process.

A preferred embodiment (40) concretizing any one of embodiments (1) to (39), wherein the process is conducted as a batch process, and wherein in (iii) the contacting of the catalyst with the reaction mixture prepared in (ii) is conducted at a temperature in the range of from 35 to 250 °C, preferably from 40 to 200 °C, more preferably from 45 to 150 °C, more preferably from 50 to 100 °C, more preferably in the range of from 55 to 80 °C, and more preferably in the range of from 60 to 70 °C.

A preferred embodiment (41) concretizing any one of embodiments (1) to (40), wherein the process is conducted as a batch process, and wherein in (iii) the contacting of the catalyst with the reaction mixture prepared in (ii) is conducted at a pressure in the range of from 1 to 30 bar(abs), preferably in the range of from 1 to 20 bar(abs), more preferably in the range of from 1 to 15 bar(abs), more preferably in the range of from 1 to 10 bar(abs), more preferably in the range of from 1 to 5, more preferably in the range of from 1 to 3 bar(abs).

A preferred embodiment (42) concretizing any one of embodiments (1) to (41), wherein the process is conducted as a batch process, wherein contacting in (iii) is carried out for a duration in the range of from 0.1 to 24 h, preferably in the range of from 0.5 to 20 h, more preferably in the range of from 1 to 15 h, more preferably in the range of from 3 to 10 h, more preferably in the range of from 4 to 8 h, more preferably in the range of from 5 to 7 h.

A preferred embodiment (43) concretizing any one of embodiments (1) to (39), wherein the process is conducted as a continuous process, and wherein in (iii) the contacting of the catalyst with the reaction mixture prepared in (ii) is conducted at a pressure in the range of from 1 to 200 bar(abs), preferably in the range of from 20 to 150 bar(abs), more preferably in the range of from 30 to 100 bar(abs), more preferably in the range of from 40 to 80 bar(abs), more preferably in the range of from 45 to 70, more preferably in the range of from 50 to 60 bar(abs).

A preferred embodiment (44) concretizing any one of embodiments (1) to (39) and (43), wherein the process is conducted as a continuous process, and wherein contacting in (iii) is conducted at a liquid hourly space velocity (LHSV) in the range of from 0.01 to 1000 ml/(g·h), preferably from 0.05 to 500 ml/(g·h), more preferably from 0.1 to 100 ml/(g·h), more preferably from 0.3 to 10 ml/(g·h), more preferably from 0.5 to 5 ml/(g·h), more preferably from 1 to 4 ml/(g·h), more preferably from 1.2 to 3 ml/(g·h), more preferably from 1.5 to 2 ml/(g·h).

A preferred embodiment (45) concretizing any one of embodiments (1) to (39), (43) and (44), wherein the process is conducted as a continuous process, and wherein in (iii) the contacting of the catalyst with the reaction mixture prepared in (ii) is conducted at a temperature in the range of from 0 to 150 °C, preferably from 5 to 100 °C, more preferably from 10 to 50 °C, more preferably from 15 to 40 °C, more preferably in the range of from 20 to 35 °C, and more preferably in the range of from 25 to 30 °C.

A preferred embodiment (46) concretizing any one of embodiments (1) to (46), wherein the reaction product obtained in (iii) further comprises water, wherein the reaction product obtained in (iii) preferably contains water in an amount in the range of from 0 to 10 wt.-%, more preferably in the range of from 1 to 8 wt-%, more preferably in the range of from 2 to 6 wt.-%, more preferably in the range of from 2.5 to 4.5 wt.-%, more preferably in the range of from 2.9 to 3.8 wt.-%, more preferably in the range of from 3.0 to 3.7 wt.-%, based on 100 wt.-% of the reaction product.

A preferred embodiment (47) concretizing any one of embodiments (1) to (46), wherein the process further comprises

(iv) separating 2,2,4,6,6-pentamethyl-1,2,5,6-tetrahydro-pyrimidine from the reaction product to obtain 2,2,4,6,6-pentamethyl-1,2,5,6-tetrahydro-pyrimidine and a residual mixture;
(v) recycling at least a portion of the residual mixture in the reaction mixture in (ii),
wherein preferably after (iv) and prior to (v) compounds having a boiling point higher than that of 2,2,4,6,6-pentamethyl-1,2,5,6-tetrahydro-pyrimidine were separated from the residual mixture.

A preferred embodiment (48) concretizing any one of embodiments (1) to (47), wherein in the reaction mixture prepared in (ii), the molar ratio of acetone to ammonia is in the range of from 1:1 to 25:1, preferably of from 2:1 to 22:1, more preferably of from 4:1 to 20:1, more preferably of from 6:1 to 18:1, more preferably of from 8:1 to 17:1, more preferably of from 10:1 to 16:1, more preferably in the range of from 12:1.0 to 14.7:1.0, more preferably in the range of from 14.2:1.0 to 14.5:1.0.

A preferred embodiment (49) concretizing any one of embodiments (1) to (48), wherein the catalyst is provided as a molding, wherein the molding is prepared according to a process comprising

(a) preparing a mixture comprising the zeolitic material and optionally one or more binders;
(b) shaping the mixture, preferably by extruding to obtain a shaped material;
(c) optionally drying the shaped material in a gas atmosphere;
(d) calcining the shaped material obtained from (b) or (c) in a gas atmosphere to obtain the molding.

A preferred embodiment (50) concretizing embodiment (49), wherein the mixture comprises one or more binders, wherein the one or more binders is one or more of alumina, silica, silica-alumina, graphite, a polysaccharide, a sugar alcohol and a synthetic polymer, preferably one or more of alumina, silica, silica-alumina, graphite, a sugar alcohol, a synthetic polymer, cellulose, a modified cellulose and a starch, more preferably silica, alumina, silica-alumina, graphite, a sugar alcohol, a synthetic polymer, a microcrystalline cellulose, a cellulose ether, more preferably graphite, sorbitol, mannitol, polyethylene glycol (PEG), polyvinylpyrrolidone (PVP), hydroxypropyl cellulose (HPC), hydroxypropyl methylcellulose (HPMC), and sodium carboxymethyl cellulose.

A preferred embodiment (51) concretizing embodiment (49) or (50), wherein in the mixture according to (a), the weight ratio of the one or more binders relative to the zeolitic material is in the range of from 1:10 to 1:30, preferably in the range of from 1:15 to 1:25.

A preferred embodiment (52) concretizing any one of embodiments (49) to (51), wherein the process comprises drying according to (c), wherein drying is conducted in a gas atmosphere having a temperature in the range of from 90 to 150 °C, preferably at a temperature in the range of from 110 to 130 °C, wherein preferably the drying is conducted for 10 to 15 h, more preferably for 11 to 13 h.

A preferred embodiment (53) concretizing any one of embodiments (49) to (52), wherein the gas atmosphere in (d) has a temperature in the range of from 400 to 600 °C, preferably in the range of from 450 to 550 °C, wherein preferably the calcining is conducted for 3 to 7 h, more preferably for 4 to 6 h.

A preferred embodiment (54) concretizing any one of embodiments (49) to (53), wherein the gas atmosphere in one or more of (c) and (d) comprises one or more of nitrogen, oxygen, and argon, preferably nitrogen, wherein preferably the gas atmosphere in one or more of (c) and (d) comprises air, wherein more preferably the gas atmosphere in one or more of (c) and (d) consists of air.

EXPERIMENTAL SECTION

The present invention is further illustrated by the following Reference Examples, Examples, and Comparative Examples.

**Reference Example 1:** Determination of the BET specific surface area, the Langmuir specific surface area, the micropore volume, the average pore width and the average pore diameter ($N_2$)

The BET specific surface area, the Langmuir specific surface area, the micropore volume, the average pore width and the average pore diameter ($N_2$) were determined via nitrogen physisorption at 77 K according to the method disclosed in DIN 66131.

**Reference Example 2:** Determination of the acid sites: Temperature programmed desorption of ammonia ($NH_3$-TPD)

The temperature-programmed desorption of ammonia ($NH_3$-TPD) was conducted in an automated chemisorption analysis unit (Micromeritics AutoChem II 2920) having a thermal conductivity detector. Continuous analysis of the desorbed species was accomplished using an online mass spectrometer (OmniStar QMG200 from Pfeiffer Vacuum). The sample (0.1 g) was introduced into a quartz tube and analysed using the program described below. The temperature was measured by means of a Ni/Cr/Ni thermocouple immediately above the sample in the quartz tube. For the analyses, He of purity 5.0 was used. Before any measurement, a blank sample was analysed for calibration.

1. Preparation: Commencement of recording; one measurement per second. Wait for 10 minutes at 25 °C and a He flow rate of 30 $cm^3$/min (room temperature (about 25 °C) and 1 atm); heat up to 600 °C at a heating rate of 20 K/min; hold for 10 minutes. Cool down under a He flow (30 $cm^3$/min) to 100 °C at a cooling rate of 20 K/min (furnace ramp temperature); Cool down under a He flow (30 $cm^3$/min) to 100 °C at a cooling rate of 3 K/min (sample ramp temperature).
2. Saturation with $NH_3$: Commencement of recording; one measurement per second. Change the gas flow to a mixture of 10 % $NH_3$ in He (75 $cm^3$/min; 100 °C and 1 atm) at 100 °C; hold for 30 min.
3. Removal of the excess: Commencement of recording; one measurement per second. Change the gas flow to a He flow of 75 $cm^3$/min (100 °C and 1 atm) at 100 °C; hold for 60 min.
4. $NH_3$-TPD: Commencement of recording; one measurement per second. Heat up under a He flow (flow rate: 30 $cm^3$/min) to 600 °C at a heating rate of 10 K/min; hold for 30 min.
5. End of measurement.

Desorbed ammonia was measured by means of the online mass spectrometer, which demonstrated that the signal from the thermal conductivity detector was caused by desorbed ammonia. This involved utilizing the m/z = 16 signal from ammonia in order to monitor the desorption of the ammonia. The amount of ammonia adsorbed (mmol/g of sample) was ascertained by means of the Micromeritics software through integration of the TPD signal with a horizontal baseline.

**Reference Example 3:** X-ray powder diffraction and determination of the crystallinity

Powder X-ray diffraction (PXRD) data was collected using a diffractometer (D8 Advance Series II, Bruker AXS GmbH) equipped with a LYNXEYE detector operated with a Copper anode X-ray tube running at 40kV and 40mA. The geometry was Bragg-Brentano, and air scattering was reduced using an air scatter shield.

Computing crystallinity for TS-1: The crystallinity calculations were performed using a calibration routine. Various mixtures of an ideally crystallized sample with the dried initial crystallization suspension without seeding crystals are created in which the crystalline material has a mass percent of 30%, 40%, 50%, 60%, 70%, 80%, 90% and 100%. These samples were measured as described below. The analysis was performed by determining the net intensities between the diffraction angles of 21,3 °2Theta and 25 °2Theta. This is performed using the "area" tool within DIFFRAC.EVA provided by Bruker AXS GmbH, Karlsruhe (User Manual for DIFFRAC.EVA Version 5, April 2019, Bruker AXS GmbH, Karlsruhe).

$$C_r = I_{sample}/I_{standard}$$

Where $I_{sample}$ is the net intensity of the sample and $I_{standard}$ is the net intensity of the standard. The raw crystallinity value was determined by calculating the net area of the sample divided by the net area of the standard sample. This is corrected by the linear calibration function.

$$c = c_r * a + b$$

Where a and b are the coefficients determined using the calibration data.

Computing crystallinity for all other zeolite samples besides TS-1: The crystallinity of the samples was determined using the software DIFFRAC.EVA provided by Bruker AXS GmbH, Karlsruhe, according to the method is described on page 121 of the user manual. The default parameters for the calculation were used.

Computing phase composition: The phase composition was computed against the raw data using the modelling software DIFFRAC.TOPAS provided by Bruker AXS GmbH (User Manual for DIFFRAC.TOPAS Version 6, 2017, Bruker AXS GmbH, Karlsruhe). The crystal structures of the identified phases, instrumental parameters as well the crystallite size of the individual phases were used to simulate the diffraction pattern. This was fit against the data in addition to a function modelling the background intensities.

Data collection: The samples were homogenized in a mortar and then pressed into a standard flat sample holder provided by Bruker AXS GmbH for Bragg-Brentano geometry data collection. The flat surface was achieved using a glass plate to compress and flatten the sample powder. The data was collected from the angular range 2 to 70 °2Theta with a step size of 0.02 °2Theta, while the variable divergence slit was set to an angle of 0.1 °. The crystalline content describes the intensity of the crystalline signal to the total scattered intensity.

**Example 1:** Preparation of H-ZSM-5 (MFI-type framework structure)

In a 2 m$^3$ reactor 79.61 kg of de-ionised water is first introduced. To the water, 411.15 kg of an aqueous tetrapropylammonium hydroxide solution (TPAOH; 40 wt. %) was added under stirring (70 rpm). The suspension is let for stirring for another 10 min. 8.2 kg solid NaOH is added slowly in 2.5 kg portions under stirring and after each portion the system is allowed to mix for 5 minutes. Next, 29.25 kg aluminium triisopropoxide is added to the suspension and the system is stirred for another 1 h. At the end, 538,19 kg colloidal silica (Ludox AS-40) is added followed by additional 10 kg of de-ionized water. The synthesis mixture is stirred another 1 h at room temperature before the reactor is flushed with nitrogen gas and the pressure reduced to 900 mbar.

Afterwards the reactor is heated to 170 °C in 11 h. The hydrothermal synthesis is run for 72 h at 170 °C under 70 rpm stirring. After crystallization the synthesis mixture is cooled down to 30 °C. The suspension is transferred to a larger vessel where the pH of the suspension is adjusted to 7 ± 0,5, by addition of a 10 wt. % aqueous nitric acid solution. The pH adjusted suspension is let for stirring for another 30 min at 70 rpm. The zeolite is separated by filtration and the filter cake is washed with de-ionised water until a conductivity of the wash water < 200 $\mu$S. The filtercake is then dried at 120 °C for 96 h. The dried material was calcined to 550 °C in air for 6 h for obtaining a calcined ZSM-5 zeolite with a BET surface area of 390 m$^2$/g, and displaying a crystallinity as determined by X-ray diffraction of 94 %.

250 kg de-ionized water is added to a 400 L reactor and 25 kg ammonium nitrate is added under stirring (150 rpm). The suspension is heated to 80 °C, followed by the addition of 25 kg of the calcined zeolite. The mixture is stirred further for 1 h at 80 °C. Afterwards the reaction mixture is cooled down and filtered off using a filterpress and washed with water until a conductivity in the wash water < 200 $\mu$S. The ion-exchange process is then repeated for obtaining an ammonium-exchanged ZSM-5. The filter cake obtained after the second ammonium ion-exchange process is dried for 10 h at 120 °C and calcined at 500 °C in air for 5 h (heating rate 2 °C/min) for obtaining H-ZSM-5.

According to the elemental analysis the resulting product had the following contents determined per 100 g substance of < 0.1 g carbon, 1.6 g aluminum, < 0.01 g of sodium, and 43 g silicon.

The BET surface area was determined to be 408 m$^2$/g.

**Example 2:** Preparation of La-ZSM-5 by wet impregnation and extrusion thereof

100 g of H-ZSM-5 as obtained according to Example 1 were added to a solution of 16.4 g of La(NO$_3$)$_3$ * 6 H$_2$O dissolved in 100 ml of distilled water and the mixture was stirred at room temperature for 2 h, after which the mixture was heated to 50 °C and evaporated to dryness over night in a rotary evaporator. The solid residue was then heated to 500 °C at a rate of 2° C/min and calcined at that temperature for 5 h for obtaining 106 g of La-ZSM-5.

The BET surface area was determined to be 318 m$^2$/g.

The 79.5 g La-ZSM-5 was then admixed with 22.08 g of colloidal silica (Ludox AS-40) and 3.9 g Walocel binder (Wolf Walsrode AG PUFAS Werk KG), wherein the resulting mixture was kneaded for 10 min, after which 50 ml of distilled water were added and the resulting mixture was kneaded for an additional 20 min. The kneaded mixture was than extruded to strands with a diameter of 2mm. The extrudate was then heated to 120 °C at a rate of 3 °C/min, held at that temperature for 7 hours, and then heated further to 500 °C at a rate of 2 °C/min and calcined at that temperature for 2 h for obtaining 66.2 g of the calcined extrudate.

**Examples 3A and 3B:** Preparation of TS-1 and extrusion thereof

3A. A TS-1 material in powder form was prepared in accordance with the experimental procedure disclosed in WO 2011/064191 A1.

3B. An extruded TS-1 material was produced in accordance with the experimental procedure disclosed in WO 2011/064191 A1.

**Example 4:** Preparation of Sn-FER

6.11 g of Sn(OAc)$_2$ were mixed with 112.5 g of deionized water to form a grey suspension. 150 g of HN$_4$-FER (CP 914 from Zeolyst) having an SAR of 20 were then added to the suspension which was then mixed using a spatula. The mixture was dried over night in a vacuum drying oven at 60 °C. The resulting powder was calcined at 500 °C for 3 h (heating ramp: 2 °C/min) for obtaining the Sn-FER product.

**Comparative Example 5:** H-zeolite Y

As Example 5, a commercial H-zeolite Y was employed (DY-32 from Evertruth) displaying a silica to alumina molar ratio of 25, and a crystallinity of 95%, wherein elemental analysis afforded 3.1wt% Al, and 41.0wt% Si. The material furthermore displayed a BET surface area of 788 m$^2$/g, and a Langmuir surface area of 1,030 m$^2$/g, a total micropore volume of 0.297 mL/g, and average pore width of 2.56 nm, an average pore diameter (N2) of 6.70 nm.

**Example 6:** Characteristics of the zeolitic materials

The respective catalytic materials from examples 2, 3B, 4 and comparative example 5 were analyzed with regard to their acid properties using temperature programmed desorption of ammonia (NH$_3$-TPD) according to reference example 2. The results are shown in table 1 below, wherein it is apparent that the materials of examples 2, 3B and 4 are distinguished from the material of comparative example 5 by a substantially smaller concentration of medium and strong acid sites.

Table 1: Acidity characteristics of the zeolitic materials from Examples 2, 3B and 4 and Comparative Example 5 as determined from NH$_3$-TPD after deconvolution.

| Catalyst | Metal (wt.-%) | Total Acid Sites [mmol/g] | Weak Acid Sites ($T_{max}$ [°C]; concentration [mmol/g]) | Medium Acid Sites ($T_{max}$ [°C]; concentration [mmol/g]) | Strong Acid Sites ($T_{max}$ [°C]; concentration [mmol/g]) |
|---|---|---|---|---|---|
| Ex 2* | La (4.9) | 0.677 | 215 ; 0.400 | 399 ; 0.265 | 564 ; 0.012 |
| Ex 3B* | Ti | 0.030 | 169 ; 0.019 | 305; 0.006 | 595 ; 0.005 |
| Ex 4 | Sn (2) | 0.840 | 204 ; 0.328 | 459 ; 0.489 | 567 ; 0,023 |
| CE 5 | - | 1.099 | 193 ; 0.285 | 368; 0.743 | 563 ; 0.071 |
| *Analytics describe catalyst's composition after extrusion. | | | | | |

**Example 7:** Catalytic testing - batch mode

Powder catalysts from examples 2, 3A and 4 and from comparative example 5 have been tested as catalysts for the production of 2,2,4,6,6-pentamethyl-1,2,5,6-tetrahydro-pyrimidine in batch mode. To this effect, the respective catalyst sample was placed in a reactor together with acetone and ammonia, wherein the catalysts were tested as the powder and not in the extruded form. The tests were run at a reactor temperature of 65°C and at a reactor pressure of 1.0-2.5 bar, wherein the duration of the test was 6 h. During the tests, the reaction mixture was stirred at a rate of 150 rpm. Before the reaction, the acetone and ammonia were present in the reactor at an acetone : NH$_3$ molar ratio of 14.30 : 1.00. The reaction product was analyzed with gas chromatography in order to determine its contents of 2,2,4,6,6-pentamethyl-1 ,2,5,6-tetrahydro-pyrimidine, 2,2,6,6-tetramethyl-4-piperidone, 2,2,4,6-tetramethyl-2,3-dihydropyridine, and acetone, wherein the results are displayed below in table 2.

Table 2: Results for catalytic testing of different zeolites in batch mode, where the conversion towards a respective compound is given in area-% as determined by GC.

| Catalyst | wt.-%* in reaction mixture | 2,2,4,6,6-pentamethyl-1,2,5,6-tetrahydro-pyrimidine | 2,2,6,6-tetramethyl-4-piperidone | 2,2,4,6-tetramethyl-2,3-dihydropyridine | acetone |
|---|---|---|---|---|---|
| Ex 2 | 1.7 wt.-% | 12.02 | 0.79 | 0.35 | 83.99 |
| Ex 3A | 4 wt.-% | 14.64 | 0.57 | 0.67 | 78.23 |
| Ex 4 | 2.5 wt.-% | 10.51 | 0.72 | 0.32 | 85.79 |
| CE 5 | 4 wt.-% | 0.49 | 19.04 | 1.36 | 74.05 |
| * g(cat)/g(total) | | | | | |

As may be taken from the results from the catalyst testing in batch mode displayed in table 2, the catalysts according to the present invention display a very high selectivity towards 2,2,4,6,6-pentamethyl-1,2,5,6-tetrahydro-pyrimidine, whereas the comparative example offers a very low yield of the desired product. In particular, as may be taken from the results, the differences between the inventive examples and the comparative example lead to a complete reversal in the selectivities towards 2,2,4,6,6-pentamethyl-1,2,5,6-tetrahydro-pyrimidine and 2,2,6,6-tetramethyl-4-piperidone, respectively. Thus the inventive examples lead to a very high selectivity towards 2,2,4,6,6-pentamethyl-1,2,5,6-tetrahydro-pyrimidine and to a very low selectivity towards 2,2,6,6-tetramethyl-4-piperidone, whereas the comparative example leads to a very high selectivity towards 2,2,6,6-tetramethyl-4-piperidone, and to a very low concentration towards 2,2,4,6,6-pentamethyl-1,2,5,6-tetrahydro-pyrimidine. As may be taken from the comparison of the acid properties of the materials in table 1, said complete reversal in the selectivity towards 2,2,4,6,6-pentamethyl-1,2,5,6-tetrahydro-pyrimidine and 2,2,6,6-tetramethyl-4-piperidone correlates with the concentration of medium and strong acid sites, and in particular of medium acid sites in the respective materials.

Example 8: Catalytic testing - continuous mode

The catalyst of Example 3B was tested as catalyst for the production of 2,2,4,6,6-pentamethyl-1,2,5,6-tetrahydro-pyrimidine in continuous mode. A reactor with 1 m length and 8 mm diameter has been used. The temperature of the reactor wall was set to room temperature, the liquid hourly space velocities to 1.5 ml/(g*h) (Trial 1) and 3.0 ml/(g*h) (Trial 2), respectively, and the pressure to 55 bar. The feed stream contained acetone and ammonia in molar ratios of 14.3 : 1 (Trial 1) and 7.15 : 1 (Trial 2), respectively. The tests were run for 6 h (Trial 1) and 46 h (Trial 2), respectively. The starting $H_2O$ content of the reaction mixture for Trial 1 and also for Trial 2 was in the range of from 0.2 to 0.3 weight-%. For Trial 1, the $H_2O$ content of the reaction product was 3.0 weight-% shortly before the end of the catalytic testing. For Trial 2, the $H_2O$ content of the reaction product was 3.7 weight-% shortly before the end of the catalytic testing.

For the catalyst testing in the continuous mode, the respective catalysts were employed as shaped bodies (0,5 - 0,8 mm split with Ludox and 20 % $SiO_2$). The reaction product was analyzed with gas chromatography in order to determine its contents of 2,2,4,6,6-pentamethyl-1,2,5,6-tetrahydro-pyrimidine, 2,2,6,6-tetramethyl-4-piperidone, 2,2,4,6-tetramethyl-2,3-dihydropyridine, mesityl oxide, acetone, diacetone alcohol (DAA), and $CH_3C(O)CH_2C(NH_2)(CH_3)_2$ (DAAM = diacetone amine), wherein the results are displayed below in table 3.

Table 3: Results for catalytic testing of the catalyst from Example 3B in continuous mode after 6 h (Trial 1) and 6.8 h (Trial 2) of reaction, where the conversion towards a respective compound is given in area-% as determined by GC.

| Catalyst / Trial | LHSV [ml/(g*h)] | acetone : NH_3 | 2,2,4,6,6-pentamethyl-1,2,5,6-tetrahydro-pyrimidine | 2,2,6,6-tetramethyl-4-piperidone | 2,2,4,6-tetramethyl-2,3-dihydropyridine | acetone |
|---|---|---|---|---|---|---|
| E 3B / 1 | 1.5 | 14.3 : 1 | 9.87 | 0.01 | 0.15 | 89.07 |
| E 3B / 2 | 3.0 | 7.15:1 | 9.25 | 0.00 | 0.15 | 89.51 |

As may be taken from the results from the catalyst testing in continuous mode displayed in table 3, the catalyst of example 3B according to the present invention displays a high selectivity towards 2,2,4,6,6-pentamethyl-1,2,5,6-tetrahydro-pyrimidine. Compared to the results obtained in batch mode, however, the catalyst shows practically no selectivity towards 2,2,6,6-tetramethyl-4-piperidone. Accordingly, even more that the batch process, it has surprisingly been found that the inventive process affords a very high selectivity towards 2,2,4,6,6-pentamethyl-1,2,5,6-tetrahydro-pyrimidine without producing any substantial amount of the side product 2,2,6,6-tetramethyl-4-piperidone.

**Cited literature:**

- F. Cavani et al. in Journal of Molecular Catalysis A: Chemical 393 (2014), 325-332
- DE 1 276 041 B

**Claims**

1. A process for the production of 2,2,4,6,6-pentamethyl-1,2,5,6-tetrahydro-pyrimidine comprising

    (i) providing a reactor containing a catalyst comprising a zeolitic material, wherein the zeolitic material comprises $YO_2$ and optionally comprises $X_2O_3$ in its framework structure, wherein Y is a tetravalent element and X is a trivalent element;
    (ii) preparing a reaction mixture comprising acetone and ammonia;
    (iii) contacting the catalyst in the reactor with the reaction mixture prepared in (ii) for obtaining a reaction product comprising 2,2,4,6,6-pentamethyl-1,2,5,6-tetrahydro-pyrimidine;
    wherein the temperature programmed desorption of ammonia ($NH_3$-TPD) profile of the zeolitic material comprised in the catalyst provided in (i) displays one or more bands associated with medium acid sites, said one or more bands having maxima in the temperature range of from 250 to 500°C, wherein the integration of said one or more bands affords a total value of 0.5 mmol/g or less, and
    wherein the mixture prepared in (ii) and contacted with the catalyst in (iii) contains water in an amount in the range of from 0.01 to 10 wt.-% based on 100 wt.-% of the reaction mixture.

2. The process of claim 1, wherein the $NH_3$-TPD profile of the zeolitic material comprised in the catalyst provided in (i) displays one or more bands associated with medium acid sites, said one or more bands having maxima in the temperature range of from 250 to 500°C, wherein the integration of said one or more bands affords a total value of 0.45 mmol/g or less.

3. The process of claim 1 or 2, wherein the $NH_3$-TPD profile of the zeolitic material comprised in the catalyst provided in (i) displays one or more bands associated with strong acid sites, said one or more bands having maxima in the temperature range of from 510 to 800°C, wherein the integration of said one or more bands affords a total value of 0.05 mmol/g or less.

4. The process of claim 3, wherein the one or more bands associated with strong acid sites have their maxima in the temperature range of from 530 to 750°C.

5. The process of any of claims 1 to 4, wherein the integration of the bands in the $NH_3$-TPD profile of the zeolitic material comprised in the catalyst provided in (i), said bands having maxima in the temperature range of from 50 to 800°C, affords a total value of 0.9 mmol/g or less.

6. The process of claim 5, wherein the bands have their maxima in the temperature range of from 80 to 750°C.

7. The process of any of claims 1 to 6, wherein the zeolitic material has a framework structure having a maximum ring size of 10 T-atoms or less.

8. The process of any of claims 1 to 7, wherein Y is selected from the group consisting of Si, Sn, Ti, Zr, Ge, and mixtures of two or more thereof.

9. The process of any of claims 1 to 8, wherein X is selected from the group consisting of B, Al, Ga, In, and mixtures of two or more thereof.

**10.** The process of any of claims 1 to 9, wherein the zeolitic material has a $YO_2 : X_2O_3$ molar ratio in the range of from 5 to 300.

**11.** The process of any of claims 1 to 10, wherein the zeolitic material provided in (i) comprises a zeolitic material having an MFI and/or an FER framework-type structure.

**12.** The process of any of claims 1 to 11, wherein the process is conducted as a batch process or as a continuous process.

**13.** The process of any of claims 1 to 12, wherein the process further comprises

(iv) separating 2,2,4,6,6-pentamethyl-1,2,5,6-tetrahydro-pyrimidine from the reaction product to obtain 2,2,4,6,6-pentamethyl-1,2,5,6-tetrahydro-pyrimidine and a residual mixture;
(v) recycling at least a portion of the residual mixture in the reaction mixture in (ii).

**14.** The process of any of claims 1 to 13, wherein in the reaction mixture prepared in (ii), the molar ratio of acetone to ammonia is in the range of from 1:1 to 25:1.

**15.** The process of any of claims 1 to 14, wherein the catalyst is provided as a molding, wherein the molding is prepared according to a process comprising

(a) preparing a mixture comprising the zeolitic material and optionally one or more binders;
(b) shaping the mixture;
(c) optionally drying the shaped material in a gas atmosphere;
(d) calcining the shaped material obtained from (b) or (c) in a gas atmosphere to obtain the molding.

**Patentansprüche**

**1.** Verfahren zur Herstellung von 2,2,4,6,6-Pentamethyl-1,2,5,6-tetrahydropyrimidin, umfassend

(i) Bereitstellen eines Reaktors, der einen Katalysator enthält, der ein zeolithisches Material umfasst, wobei das zeolithische Material $YO_2$ und gegebenenfalls $X_2O_3$ in seiner Gerüststruktur umfasst, wobei Y für ein vierwertiges Element steht und X für ein dreiwertiges Element steht;
(ii) Herstellen einer Reaktionsmischung, die Aceton und Ammoniak umfasst;
(iii) Inkontaktbringen des Katalysators in dem Reaktor mit der in (ii) hergestellten Reaktionsmischung unter Erhalt eines Reaktionsprodukts, das 2,2,4,6,6-Pentamethyl-1,2,5,6-tetrahydropyrimidin umfasst;
wobei das Profil der temperaturprogrammierten Desorption von Ammoniak ($NH_3$-TPD) des in dem in (i) bereitgestellten Katalysator enthaltenen zeolithischen Materials eine oder mehrere Banden, die mit mittelsauren Zentren assoziiert ist bzw. sind, umfasst, wobei die eine oder die mehreren Banden Maxima im Temperaturbereich von 250 bis 500 °C aufweist bzw. aufweisen, wobei die Integration der einen oder mehreren Banden einen Gesamtwert von 0,5 mmol/g oder weniger liefert, und
wobei die in (ii) hergestellte und in (iii) mit dem Katalysator in Kontakt gebrachte Mischung Wasser in einer Menge im Bereich von 0,01 bis 10 Gew.-%, bezogen auf 100 Gew.-% der Reaktionsmischung, enthält.

**2.** Verfahren nach Anspruch 1, wobei das $NH_3$-TPD-Profil des in dem in (i) bereitgestellten Katalysator enthaltenen zeolithischen Materials eine oder mehrere Banden, die mit mittelsauren Zentren assoziiert ist bzw. sind, umfasst, wobei die eine oder die mehreren Banden Maxima im Temperaturbereich von 250 bis 500 °C aufweist bzw. aufweisen, wobei die Integration der einen oder mehreren Banden einen Gesamtwert von 0,45 mmol/g oder weniger liefert.

**3.** Verfahren nach Anspruch 1 oder 2, wobei das $NH_3$-TPD-Profil des in dem in (i) bereitgestellten Katalysator enthaltenen zeolithischen Materials eine oder mehrere Banden, die mit stark sauren Zentren assoziiert sind, umfasst, wobei die eine oder die mehreren Banden Maxima im Temperaturbereich von 510 bis 800 °C aufweist bzw. aufweisen, wobei die Integration der einen oder mehreren Banden einen Gesamtwert von 0,05 mmol/g oder weniger liefert.

**4.** Verfahren nach Anspruch 3, wobei die eine oder die mehreren Banden, die mit stark sauren Zentren assoziiert ist bzw. sind, ihre Maxima im Temperaturbereich von 530 bis 750 °C aufweist bzw. aufweisen.

**5.** Verfahren nach einem der Ansprüche 1 bis 4, wobei die Integration der Banden im $NH_3$-TPD-Profil des in dem in (i) bereitgestellten Katalysator enthaltenen zeolithischen Materials, wobei die Banden Maxima im Temperaturbereich von 50 bis 800 °C aufweisen, einen Gesamtwert von 0,9 mmol/g oder weniger liefert.

**6.** Verfahren nach Anspruch 5, wobei die Banden ihre Maxima im Temperaturbereich von 80 bis 750 °C aufweisen.

**7.** Verfahren nach einem der Ansprüche 1 bis 6, wobei das zeolithische Material eine Gerüststruktur mit einer maximalen Ringgröße von 10 T-Atomen oder weniger aufweist.

**8.** Verfahren nach einem der Ansprüche 1 bis 7, wobei Y aus der Gruppe bestehend aus Si, Sn, Ti, Zr, Ge und Mischungen von zwei oder mehr davon ausgewählt ist.

**9.** Verfahren nach einem der Ansprüche 1 bis 8, wobei X aus der Gruppe bestehend aus B, Al, Ga, In und Mischungen von zwei oder mehr davon ausgewählt ist.

**10.** Verfahren nach einem der Ansprüche 1 bis 9, wobei das zeolithische Material ein $YO_2 : X_2O_3$-Molverhältnis im Bereich von 5 bis 300 aufweist.

**11.** Verfahren nach einem der Ansprüche 1 bis 10, wobei das in (i) bereitgestellte zeolithische Material ein zeolithisches Material mit einer MFI-und/oder FER-Gerüsttyp-Struktur aufweist.

**12.** Verfahren nach einem der Ansprüche 1 bis 11, wobei das Verfahren als diskontinuierliches Verfahren oder als kontinuierliches Verfahren durchgeführt wird.

**13.** Verfahren nach einem der Ansprüche 1 bis 12, wobei das Verfahren ferner Folgendes umfasst:

(iv) Abtrennen von 2,2,4,6,6-Pentamethyl-1,2,5,6-tetrahydropyrimidin aus dem Reaktionsprodukt unter Erhalt von 2,2,4,6,6-Pentamethyl-1,2,5,6-tetrahydropyrimidin und einer Restmischung;
(v) Zurückführen mindestens eines Teils der Restmischung in die Reaktionsmischung in (ii).

**14.** Verfahren nach einem der Ansprüche 1 bis 13, wobei in der in (ii) hergestellten Reaktionsmischung das Molverhältnis von Aceton zu Ammoniak im Bereich von 1:1 bis 25:1 liegt.

**15.** Verfahren nach einem der Ansprüche 1 bis 14, wobei der Katalysator als Formkörper bereitgestellt wird, wobei der Formkörper gemäß einem Verfahren hergestellt wird, das Folgendes umfasst:

(a) Herstellen einer Mischung, die das zeolithische Material und gegebenenfalls ein oder mehrere Bindemittel umfasst;
(b) Formen der Mischung;
(c) gegebenenfalls Trocknen des geformten Materials in einer Gasatmosphäre;
(d) Calcinieren des aus (b) bzw. (c) erhaltenen geformten Materials in einer Gasatmosphäre unter Erhalt des Formkörpers.

## Revendications

**1.** Procédé pour la production de 2,2,4,6,6-pentaméthyl-1,2,5,6-tétrahydro-pyrimidine comprenant

(i) la fourniture d'un réacteur contenant un catalyseur comprenant un matériau zéolithique, le matériau zéolithique comprenant $YO_2$ et comprenant éventuellement $X_2O_3$ dans sa structure de cadre, Y étant un élément tétravalent et X étant un élément trivalent ;
(ii) préparation d'un mélange réactionnel comprenant de l'acétone et de l'ammoniac ;
(iii) mise en contact du catalyseur dans le réacteur avec le mélange réactionnel préparé en (ii) pour obtenir un produit de réaction comprenant de la 2,2,4,6,6-pentaméthyl-1,2,5,6-tétrahydro-pyrimidine ;
le profil de désorption programmée en température de l'ammoniac ($NH_3$-TPD) du matériau zéolithique compris dans le catalyseur fourni en (i) présentant une ou plusieurs bandes associées à des sites d'acide moyen, ladite ou lesdites bandes ayant des maximums dans la plage de températures allant de 250 à 500 °C, l'intégration de ladite ou desdites bandes fournissant une valeur totale de 0,5 mmole/g ou moins, et
le mélange préparé en (ii) et mis en contact avec le catalyseur en (iii) contenant de l'eau en une quantité dans la plage allant de 0,01 à 10 % en poids sur la base de 100 % en poids du mélange réactionnel.

2. Procédé selon la revendication 1, le profil $NH_3$-TPD du matériau zéolithique compris dans le catalyseur fourni en (i) présentant une ou plusieurs bandes associées à des sites d'acide moyen, ladite ou lesdites bandes ayant des maximums dans la plage de températures allant de 250 à 500 °C, l'intégration de ladite ou desdites bandes fournissant une valeur totale de 0,45 mmole/g ou moins.

3. Procédé selon la revendication 1 ou 2, le profil $NH_3$-TPD du matériau zéolithique compris dans le catalyseur fourni en (i) présentant une ou plusieurs bandes associées à des sites d'acide fort, ladite ou lesdites bandes ayant des maximums dans la plage de températures allant de 510 à 800 °C, l'intégration de ladite ou desdites bandes fournissant une valeur totale de 0,05 mmole/g ou moins.

4. Procédé selon la revendication 3, la ou les bandes associées à des sites d'acide fort ayant leurs maximums dans la plage de températures allant de 530 à 750 °C.

5. Procédé selon l'une quelconque des revendications 1 à 4, l'intégration des bandes dans le profil $NH_3$-TPD du matériau zéolithique compris dans le catalyseur fourni en (i), lesdites bandes ayant des maximums dans la plage de températures allant de 50 à 800 °C, fournissant une valeur totale de 0,9 mmole/g ou moins.

6. Procédé selon la revendication 5, les bandes ayant leurs maximums dans la plage de températures allant de 80 à 750 °C.

7. Procédé selon l'une quelconque des revendications 1 à 6, le matériau zéolithique ayant une structure de cadre ayant une taille maximale de cycle de 10 atomes T ou moins.

8. Procédé selon l'une quelconque des revendications 1 à 7, Y étant choisi dans le groupe constitué par Si, Sn, Ti, Zr, Ge, et des mélanges de deux ou plus de ceux-ci.

9. Procédé selon l'une quelconque des revendications 1 à 8, X étant choisi dans le groupe constitué par B, Al, Ga, In, et des mélanges de deux ou plus de ceux-ci.

10. Procédé selon l'une quelconque des revendications 1 à 9, le matériau zéolithique ayant un rapport molaire $YO_2$: $X_2O_3$ dans la plage allant de 5 à 300.

11. Procédé selon l'une quelconque des revendications 1 à 10, le matériau zéolithique fourni en (i) comprenant un matériau zéolithique ayant une structure de type de cadre MFI et/ou une structure de type de cadre FER.

12. Procédé selon l'une quelconque des revendications 1 à 11, le procédé étant conduit comme un procédé en lot ou comme un procédé continu.

13. Procédé selon l'une quelconque des revendications 1 à 12, le procédé comprenant en outre

(iv) la séparation de 2,2,4,6,6-pentaméthyl-1,2,5,6-tétrahydro-pyrimidine du produit de réaction pour obtenir de la 2,2,4,6,6-pentaméthyl-1,2,5,6-tétrahydro-pyrimidine et un mélange résiduel ;
(v) le recyclage d'au moins une partie du mélange résiduel dans le mélange réactionnel en (ii).

14. Procédé selon l'une quelconque des revendications 1 à 13, dans le mélange réactionnel préparé en (ii), le rapport molaire d'acétone sur ammoniac étant dans la plage allant de 1:1 à 25:1.

15. Procédé selon l'une quelconque des revendications 1 à 14, le catalyseur étant fourni comme un moulage, le moulage étant préparé selon un procédé comprenant

(a) la préparation d'un mélange comprenant le matériau zéolithique et éventuellement un ou plusieurs liants ;
(b) la mise en forme du mélange ;
(c) éventuellement le séchage du matériau façonné dans une atmosphère de gaz ;
(d) la calcination du matériau façonné obtenu de (b) ou (c) dans une atmosphère de gaz pour obtenir le moulage.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 1276041 B

- WO 2011064191 A1

**Non-patent literature cited in the description**

- **CAVANI et al.** *Journal of Molecular Catalysis A: Chemical,* 2014, vol. 393, 325-332

- **F. CAVANI et al.** *Journal of Molecular Catalysis A: Chemical,* 2014, vol. 393, 325-332